# EUROPEAN PATENT APPLICATION

(11) **EP 3 451 689 A1**
(43) Date of publication of application: **06.03.2019**
(21) Application number: 17188872.0
(22) Date of filing: 31.08.2017
(51) Int. Cl.: H04R 1/10

(54) **CONTROL INTERFACE FOR HEARING PROTECTION HEADSET**

(71) Applicant: Honeywell International Inc., Morris Plains, NJ 07950 (US)
(72) Inventor: WILSON, May, Morris Plains, NJ New Jersey 07950 (US); OHLANDER, Lisa, Morris Plains, NJ New Jersey 07950 (US); KEAN, Craig, Morris Plains, NJ New Jersey 07950 (US)
(74) Representative: Houghton, Mark Phillip

(57) **Abstract**

Embodiments relate generally to systems and methods for controlling a headset via a control interface. A headset may comprise one or more earmuffs configured to provide sound blocking; a headband connecting the earmuffs; and a control interface located on an external surface of at least one of the earmuffs, the control interface comprising no more than ten buttons, and configured to receive input from a user via at least one of the no more than ten buttons; control one or more functions of the headset based on the input from the user; and provide feedback to the user based on the received input, wherein the control interface cannot be seen by the user when the user is wearing the headset.

## Description

### CROSS-REFERENCE TO RELATED APPLICATIONS

Not applicable.

### STATEMENT REGARDING FEDERALLY SPONSORED

### RESEARCH OR DEVELOPMENT

Not applicable.

### REFERENCE TO A MICROFICHE APPENDIX

Not applicable.

### BACKGROUND

Hearing protection headsets may comprise large earmuffs attached to a headband to be worn over a wearer's head, holding the earmuffs over the wearer's ears. The earmuffs of a hearing protection headset may comply with hearing protection regulations, wherein the size, shape, and materials may be affected by the regulations. Hearing protection headsets may be worn for extended periods of time while the wearer is working in areas where hearing protection is required. Additionally, consumer headsets may comprise sound attenuation properties to prevent external noise from penetrating to a user's ear, while still allowing a user to hear sounds produced by a speaker within the headset.

### SUMMARY

In an embodiment, a control interface comprising no more than ten buttons may be configured to receive input from a user via at least one of the no more than ten buttons; control one or more functions of a headset based on the input from the user; and provide feedback to the user based on the received input, wherein the control interface cannot be seen by the user when the user is wearing the headset.

In an embodiment, a method for controlling a hearing protection headset may comprise receiving input from a user to a control interface comprising no more than ten buttons; controlling one or more functions of the headset based on the input from the user; and providing feedback to the user based on the received input, wherein the control interface cannot be seen by the user when the user is wearing the headset.

In an embodiment, a headset may comprise one or more earmuffs configured to provide sound blocking; a headband connecting the earmuffs; and a control interface located on an external surface of at least one of the earmuffs, the control interface comprising no more than ten buttons, and configured to receive input from a user via at least one of the no more than ten buttons; control one or more functions of the headset based on the input from the user; and provide feedback to the user based on the received input, wherein the control interface cannot be seen by the user when the user is wearing the headset.

### BRIEF DESCRIPTION OF THE DRAWINGS

For a more complete understanding of the present disclosure, reference is now made to the following brief description, taken in connection with the accompanying drawings and detailed description, wherein like reference numerals represent like parts.
FIG. 1 illustrates a headset comprising a control interface according to an embodiment of the disclosure.
FIG. 2 illustrates another headset comprising a control interface according to an embodiment of the disclosure.
FIG. 3 illustrates another headset comprising a control interface according to an embodiment of the disclosure.
FIG. 4 illustrates another headset comprising a control interface according to an embodiment of the disclosure.
FIG. 5 illustrates a control interface for use with a headset comprising a control interface according to an embodiment of the disclosure.
FIG. 6 illustrates another control interface for use with a headset comprising a control interface according to an embodiment of the disclosure.

### DETAILED DESCRIPTION

It should be understood at the outset that although illustrative implementations of one or more embodiments are illustrated below, the disclosed systems and methods may be implemented using any number of techniques, whether currently known or not yet in existence. The disclosure should in no way be limited to the illustrative implementations, drawings, and techniques illustrated below, but may be modified within the scope of the appended claims along with their full scope of equivalents.

The following brief definition of terms shall apply throughout the application:

The term "comprising" means including but not limited to, and should be interpreted in the manner it is typically used in the patent context;

The phrases "in one embodiment," "according to one embodiment," and the like generally mean that the particular feature, structure, or characteristic following the phrase may be included in at least one embodiment of the present invention, and may be included in more than one embodiment of the present invention (importantly, such phrases do not necessarily refer to the same embodiment);

If the specification describes something as "exemplary" or an "example," it should be understood that refers to a non-exclusive example;

The terms "about" or "approximately" or the like, when used with a number, may mean that specific number, or alternatively, a range in proximity to the specific number, as understood by persons of skill in the art field; and

If the specification states a component or feature "may," "can," "could," "should," "would," "preferably," "possibly," "typically," "optionally," "for example," "often," or "might" (or other such language) be included or have a characteristic, that particular component or feature is not required to be included or to have the characteristic. Such component or feature may be optionally included in some embodiments, or it may be excluded.

Embodiments of the disclosure include systems and methods for controlling the functions of a hearing protection device (or headset). A headset may provide hearing protection that may be required in noisy environments, while allowing the user to listen to a radio, personal listening devices, communication devices, and/or other audio sources safely at work and/or at home. It may be required and/or advisable for the user to stay protected (by wearing the headset) and/or to keep the headset on to hear important communication alarms and warning signals and co-workers' voices, for example for a substantial, continuous time (for example during a work shift). During such a period of time, a user may wish to control certain functions of the headset (for example, communication, radio, etc.) while wearing the headset, such that ideally these control functions might occur while the user simultaneously wears the headset in protective mode (e.g. over the user's ears) - for example with the headset configured to facilitate control of such functions without having to remove the headset (for example by operating control functions blindly by feel, despite the fact that the control interface is typically located on the headset (e.g. on a surface of an earmuff)). These functions may include volume control, hear-through control, communication controls, status requests, audio source controls (such as changing radio station, track number, etc.) among other similar functions.

It may be beneficial to have the control interface (e.g. for controlling the functions of the headset) located on the headset (e.g. on one or more face/surface of one or more earmuff), for example so that the control interface is always readily accessible and/or so there is no additional hardware required to control functions and/or so there is no increased risk of snags or other physical interference while a user is wearing the headset (which could raise a safety hazard). To provide logical, intuitive, error free interaction between the user and the headset (for example, without having to remove the headset and/or while the headset is in protective mode and/or blindly, for example by feel), embodiments of the disclosure may include a control interface incorporated into the headset, where the control interface may comprise a limited number of buttons or other control input elements (which may be configured to assist/guide a user in blind operation of the headset device). Operation of the control interface may be "blind" through touch (e.g. raised or recessed buttons, for example with all buttons raised, all buttons recessed, or some buttons raised and other buttons recessed), tactility (e.g. buttons having a surface with a different tactile feel (e.g. texture) than the surrounding surface of the headset and/or different buttons having different tactile feel (e.g. texture) from one another), and/or audio feedback, because the user may interact with the control interface while wearing the headset. The layout and topography of the control interface may be designed to allow simple operation and/or to direct the user toward a desired button. The control interface may be located on an external face of an earmuff of the headset. The control interface may comprise all controls for the headset, and may be positioned on one earmuff using a single printed circuit board (PCB) or other processor, thereby maintaining a compact size (for example, not substantially enlarging the earmuff over its hearing protection requirement). The control interface typically interacts with and/or operates/controls the underlying processor element(s) located within the headset (such as a PCB, storage memory, etc.), which performs the various functionality of the headset (for example by interacting with control interface, speakers, radio communication device, personal sound device, etc. incorporated into and/or interfacing with the headset - for example, receiving input signals from, processing signals, and/or generating and/or transmitting output signals to other electronic equipment within and/or used with the headset).

The control interface may comprise one or more elements for providing feedback to a user (e.g. for signaling when a button has been pressed and/or which button has been pressed and/or which function has been accessed), such as audio feedback and/or haptic feedback. The control interface may also comprise one or more indicators that may be viewed by a user when the headset is not worn, and/or the indicators may be viewed by others located near the user.

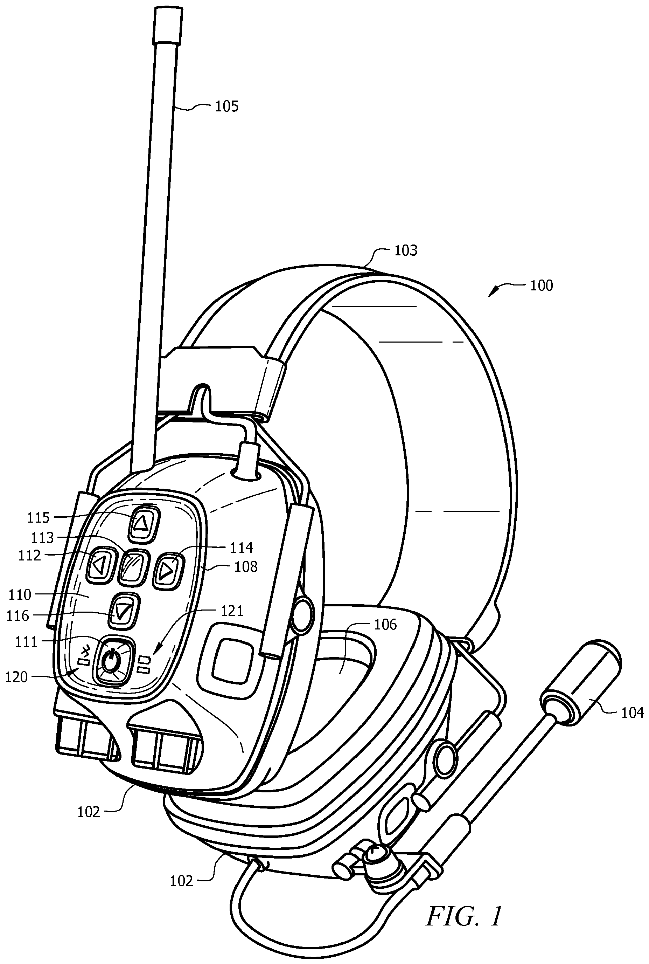

Referring now to FIG. 1, an exemplary headset 100 is shown, wherein the headset 100 comprises hearing protection earmuffs 102, a connecting head band 103 attached to the earmuffs 102, and a control interface 110 configured to receive control inputs from a user and control functions of the headset 100. In some embodiments, the headset 100 may also comprise additional communication elements, such as a microphone 104 and an antenna 105. The antenna 105 may facilitate radio communication to and from the headset 100.

The control interface 110 may comprise a plurality of buttons, and may comprise no more than ten buttons. In some embodiments, the control interface 110 may comprise no more than six buttons. The simplicity of the control interface 110 (e.g. with limited number of buttons and/or specific intuitive arrangement/layout of the buttons on the control interface which may easily be discerned via touch/feel) may allow a user to easily interact with the control interface 110 while wearing the headset 100. In other words, the control interface 110 may be configured for "blind" use, where a user does not have to view the control interface 110 to interact with it. This may allow a user to perform certain functions on the headset 100 without having to remove the headset 100, thereby ensuring that the user is protected from sound exposure (even while operating the control functions via the control interface located on the headset). In addition to the buttons located on the control interface 110, the headset 100 may comprise one or more speakers 106 configured to communicate audio feedback to a user based on the user's inputs to the control interface 110. In some embodiments, the control interface 110 may comprise a frame 108 configured to define an outer perimeter of the control interface 110. The frame 108 may indicate the location of the control interface 110 to a user. In some embodiments, the portion of the surface of the headset earmuff which forms the control interface may be easily discernable tactilely (e.g. by blind feel) and/or located within a frame/edge 108 in a way that clearly demarcates it from the remainder of the earmuff (e.g. it maybe a flat/planar surface on an otherwise curved earmuff and/or it maybe slightly recessed or raised with respect to the remainder of the earmuff and/or the frame/edge 108).

In the embodiment shown in FIG. 1, the control interface 110 may comprise a power button 111 (which in Fig. 1 is located separate and apart from (for example below the other buttons, for example directly below the down button 116), a center button 113, a left button 112, a right button 114, an up button 115, and a down button 116. The buttons may be described herein using their location relative to the center button 113; however, the description may or may not define the function of the button when pressed. In Fig. 1, the center, left, and right buttons typically may be located on a horizontal line, while the center, up, and down buttons may be located on a vertical line (e.g. with the power button also located on that vertical line). Additionally, the control interface 110 may comprise one or more indicators 120 and 121 that may be viewed by a user when not wearing the headset 100 and/or by other people located near the user.

In some embodiments, the buttons of the control interface 110 may be positioned to facilitate ease of use. In some embodiments, the buttons of the control interface 110 may comprise a specific topography to enable a user to identify the button they wish to press. In the embodiment shown in FIG. 1, the buttons may comprise a raised surface protruding from the surface of the control interface 110.

In the example shown in FIG. 1, the button layout employs appropriate hierarchies, where the most important and frequently used functions may be accessed by pressing the center button 113 (e.g. the center button may access functions which are identified as most used or most critical and/or may operate to change between menus (e.g. with different menus for different types of functions, with two or more of the other buttons (e.g. left, right, up, down) often allowing scrolling and/or selection within the selected menu to select specific functions). In some embodiments, the center button 113 may be higher (or lower and/or have a different tactile feel) than the surrounding buttons, allowing a user to (blindly) identify the center button 113 quickly (and then to base operation of the other buttons from that central location). Additionally, the power button 111 (which may also function as a wireless pairing button in some embodiments, for example with the headset automatically searching for any devices with which to pair upon power-up) may be placed in a lower position on the control interface, separate from the buttons 112, 113, 114, 115, 116 designated for system control, thereby creating a clear hierarchy in the user's mind (e.g. based on button layout). In some embodiments, the power button 111 may be larger in size and/or differently shaped than the other buttons to represent its importance and/or different function (e.g. so the user will not inadvertently turn off the headset while trying to operate control functions).

The headset 100 may comprise a plurality of communication capabilities, which may all be controlled using the control interface 110. The limited number of buttons may complete all functions necessary to utilize the features of the headset 100. As an example, the headset 100 may comprise one or more of Bluetooth communication, wireless communication, wired communication, radio communication, hear-through communication, local device communication, etc.

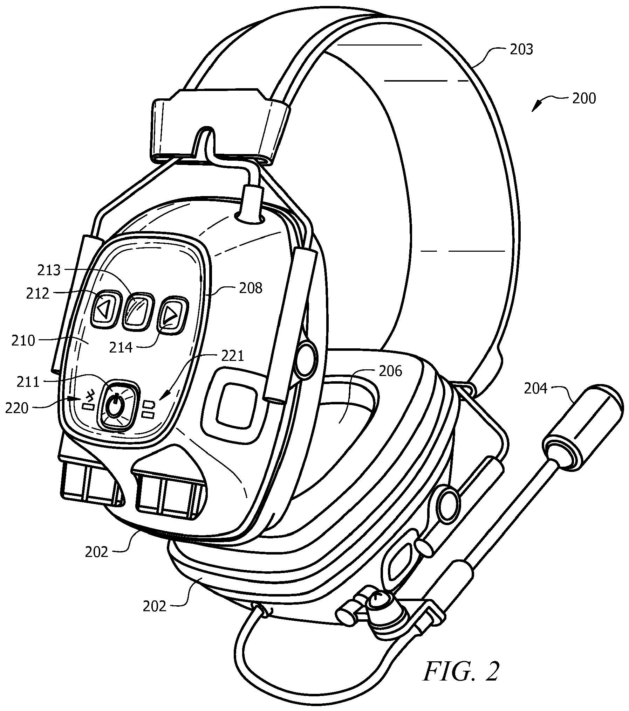

FIG. 2 illustrates an embodiment of a headset 200 (which may be similar to the headset 100 described above), where the headset 200 may not comprise radio communication capabilities. This may reduce the number of functions that may be controlled by a control interface 210, and therefore the headset 200 may comprise no more than four buttons. The headset 200 may comprise the earmuffs 202, headband 203, speaker 206, and optional microphone 204. The control interface 210 of the headset 200 may comprise a frame 208, a power button 211, a center button 213, a left button 212, and a right button 214. The buttons may be described herein using their location relative to the center button 213; however, the description may or may not define the function of the button when pressed. Additionally, the control interface 210 may comprise one or more indicators 220 and 221 that may be viewed by a user when not wearing the headset 200 and/or by other people located near the user. The control interface 210 may be similar to the control interface 110 described above. The topography and layout of the control interface 210 may be similar to the control interface 110 described above, excluding the buttons that are not shown in FIG. 2 (e.g. the up and down buttons, although in other embodiments the control interface might include up and down buttons instead of left and right buttons).

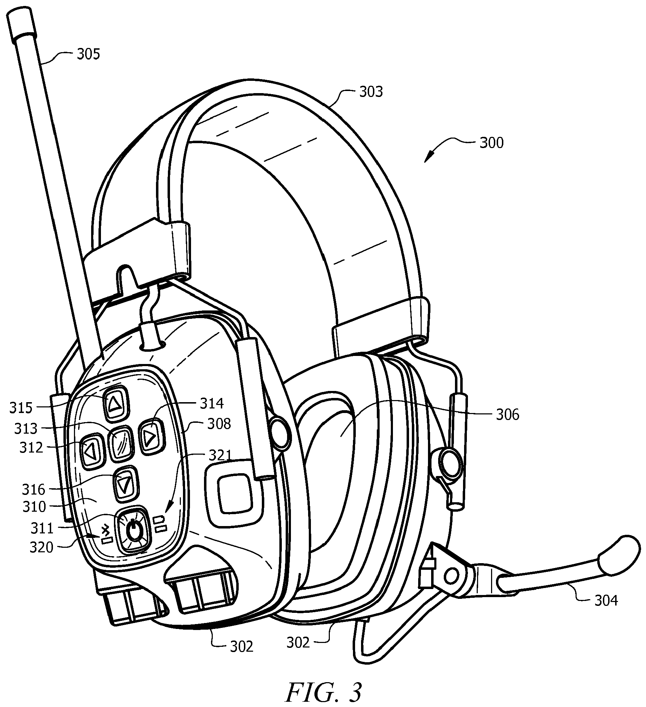

FIG. 3 illustrates an embodiment of a headset 300 that is similar to the headset 100 described above. However, the topography of the buttons of the control interface 310 may be different. The headset 300 may comprise the earmuffs 302, headband 303, speaker 306, optional microphone 304, and optional antenna 305. The control interface 310 of the headset 300 may comprise a frame 308, a power button 311, a center button 313, a left button 312, a right button 314, an up button 315, and a down button 316. The buttons may be described herein using their location relative to the center button 313; however, the description may or may not define the function of the button when pressed. Additionally, the control interface 310 may comprise one or more indicators 320 and 321 that may be viewed by a user when not wearing the headset 300 and/or by other people located near the user. The control interface 310 may be similar to the control interface 110 described above.

To define the positions of the buttons for a user, the center button 313 may be recessed while the surrounding buttons 312, 314, 315, 316 may be elevated or raised from the surface of the control interface 310. Additionally, the power button 311 may be recessed, to indicate the significance and/or different purpose of that button.

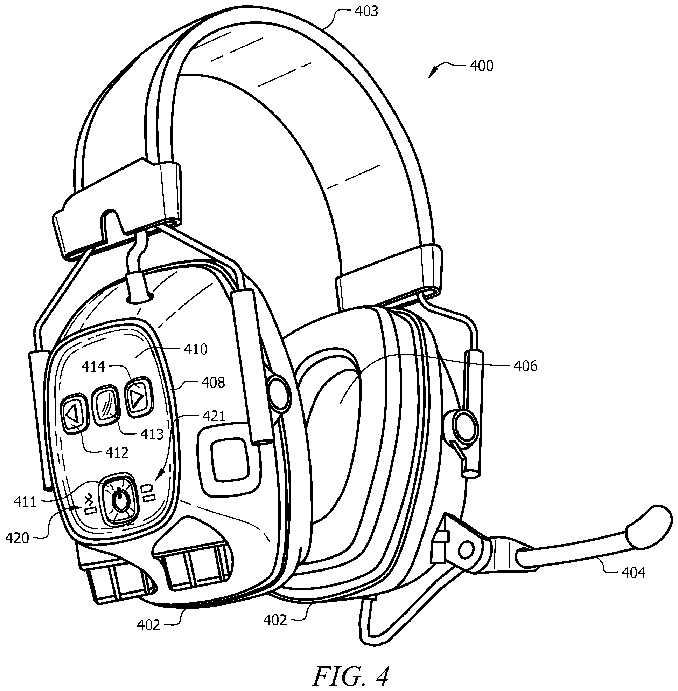

FIG. 4 illustrates an embodiment of a headset 400 (which may be similar to the headset 300 and/or 200 described above), where the headset 400 may not comprise radio communication capabilities. This may reduce the number of functions that may be controlled by a control interface 410, and therefore the headset 400 may comprise no more than four buttons. The headset 400 may comprise the earmuffs 402, headband 403, speaker 406, and optional microphone 404. The control interface 410 of the headset 400 may comprise a frame 408, a power button 411, a center button 413, a left button 412, and a right button 414. The buttons may be described herein using their location relative to the center button 413; however, the description may or may not define the function of the button when pressed. Additionally, the control interface 410 may comprise one or more indicators 420 and 421 that may be viewed by a user when not wearing the headset 400 and/or by other people located near the user. The control interface 410 may be similar to the control interface 110 described above. The topography and layout of the control interface 410 may be similar to the control interface 310 described above, excluding the buttons that are not shown in FIG. 4.

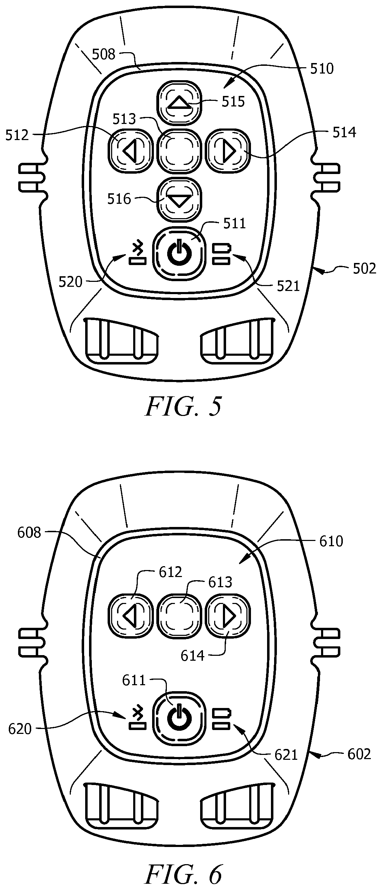

FIG. 5 illustrates a detailed view of a control interface 510 for use with a headset (simultaneously and as described above, for example with respect to Figs. 1 and/or 3). As described above, the control interface 510 may be located on an external face of an earmuff 502. The control interface 510 may comprise a frame 508 located around and defining the perimeter of the control interface 510. The frame/edge 508 may be at a height different than that of the remainder of the control interface 510 (e.g. the control interface surface), and may therefore be felt by a user's hand. In some embodiments, at least a portion of the frame 508 may be raised above the surface of the control interface 510. In some embodiments, at least a portion of the frame 508 may be recessed with respect to the control interface 510.

The control interface 510 may comprise a plurality of buttons, which may be less than ten buttons. In some embodiments, the control interface 510 may comprise no more than six buttons (e.g. six buttons as shown). As described above, the control interface 510 may comprise a power button 511 configured to power the headset off and on, and optionally configured to establish wireless communication pairing. The power button 511 may be located separately from the other buttons designated for system controls. In some embodiments, the control interface 510 may comprise one or more indicators 520 and 521 configured to indicate statuses for one or more functions of the headset. In some embodiments, the indicators 520 and 521 may comprise colored lights, such as light emitting diodes (LEDs). In the example shown in FIG. 5, a first indicator 520 may indicate Bluetooth connectivity status, and a second indicator 521 may indicate battery power remaining for the headset. In the embodiment shown in FIG. 5, the indicators 520 and 521 may be located proximate to the power button 511 (e.g. on either side of the power button).

The control interface 510 may comprise buttons 512, 513, 514, 515, and 516 designated for system controls. These buttons may comprise a center button 513, a left button 512, a right button 514, an up button 515, and a down button 516. The buttons may be described in relation to the center button 513, but these same buttons may be described in other ways, such as function and/or input.

The buttons 511, 512, 513, 514, 515, and 516 may comprise topographical characteristics configured to identify or designate the buttons for the user. For example, one or more buttons may be raised with respect to the surface of the control interface 510. As another example, one or more buttons may be raised with respect to other buttons. As another example, one or more buttons may be recessed with respect to the surface of the control interface 510. As another example, one or more buttons may be recessed with respect to other buttons.

In some embodiments, the up button 515 and the down button 516 may be designated for radio controls. In a headset that does not have radio communication capabilities, these buttons may be unnecessary, as shown in FIG. 6.

FIG. 6 illustrates a detailed view of a control interface 610 for use with a headset (as described above). As described above, the control interface 610 may be located on an external face of an earmuff 602. The control interface 610 may comprise a frame 608 located around and defining the perimeter of the control interface 610. The frame 608 may be at a height different than that of the control interface 610, and may therefore be felt by a user's hand. In some embodiments, at least a portion of the frame 608 may be raised above the surface of the control interface 610. In some embodiments, at least a portion of the frame 608 may be recessed with respect to the control interface 610.

The control interface 610 may comprise a plurality of buttons, which may be less than ten buttons. In some embodiments, the control interface 610 may comprise no more than six buttons. As described above, the control interface 610 may comprise a power button 611 configured to power the headset off and on, and optionally configured to establish wireless communication pairing. The power button 611 may be located separately from the other buttons designated for system controls. In some embodiments, the control interface 610 may comprise one or more indicators 620 and 621 configured to indicate statuses for one or more functions of the headset. In some embodiments, the indicators 620 and 621 may comprise colored lights, such as light emitting diodes (LEDs). In the example shown in FIG. 6, a first indicator 620 may indicate Bluetooth connectivity status, and a second indicator 621 may indicate battery power remaining for the headset. In the embodiment shown in FIG. 6, the indicators 620 and 621 may be located proximate to the power button 611.

The control interface 610 may comprise buttons 612, 613, and 614 designated for system controls. These buttons may comprise a center button 613, a left button 612, and a right button 614. The buttons may be described in relation to the center button 613, but these same buttons may be described in other ways, such as function and/or input.

The buttons 611, 612, 613, and 614 may comprise topographical characteristics configured to identify or designate the buttons for the user. For example, one or more buttons may be raised with respect to the surface of the control interface 610. As another example, one or more buttons may be raised with respect to other buttons. As another example, one or more buttons may be recessed with respect to the surface of the control interface 610. As another example, one or more buttons may be recessed with respect to other buttons.

The buttons of the control interface (as described above in any of FIGS. 1-6) may be configured to complete a number of functions on the headset. Table 1 below describes examples of the functions that may be completed by the one or more buttons of the control interface. Table 2 below illustrates an example of how the indicators may produce notifications for a user and/or nearby person.

**Table 1: Control Interface and Tones**

| Actions | 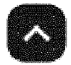 | 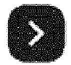 | 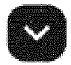 | 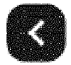 | 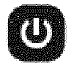 | 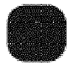 | Audio Feedback Tones |
|---|---|---|---|---|---|---|---|
| | up | right | down | left | power | center | |
| Global actions | | | | | | | |
| Power on + enter auto pairing | | | | | Hold 2s | | 4 note rising scale |
| Power off | | | | | Hold 2s | | 4 note falling scale |
| Quick pairing mode | | | | | x2 press | | 2 note rising scale |
| Mode change | | | | | press | | short 1 |

| Global (activated from any mode) | | | | | | | |
|---|---|---|---|---|---|---|---|
| Answer call | | | | | | press | ring tone stops |
| Voice Dialing | | | | | | hold 2s | short double high |
| Transfer call to external device | | | | x2 press | | | 5^{th} up short-short |
| Clear paired device list & enter manual pairing | | | | | Hold 5s | | short double low |

| Talk mode | | | | | | | |
|---|---|---|---|---|---|---|---|
| End call | | | | | | press | short double low |
| Mute microphone toggle | | press | | | | | 2 note falling scale |
| Reject incoming call | | | | press | | | short double low |

| Device playback | | | | | | | |
|---|---|---|---|---|---|---|---|
| Play | | | | | press | | short 1 |
| Pause | | | | | press | | short 1 |
| Next track | | press | | | | | short 1 |
| Scan track forward | | hold 2s | | | | | short 1 |
| Scan track backward | | | | hold 2s | | | short 1 |
| Previous track | | | | press | | | short 1 |

| FM radio | | | | | | | |
|---|---|---|---|---|---|---|---|
| Station search forward | | press | | | | | short 1 |
| Station scan forward | | hold 2s | | | | | short 1 |
| Station search backward | | | | press | | | short 1 |
| Station scan backward | | | | hold 2s | | | short 1 |
| Recall presets backward | | | press | | | | short 1 |
| Recall presets forward | press | | | | | | short 1 |
| Store station | hold 2s | | | | | | Long press, after 1 sec |
| Delete station | | | hold 2s | | | | Long press, after 1 sec |
| Mute radio toggle | | | | | | press | short 1 |

**Table 2: Global Notifications**

| **Actions** | **Tone** | **LED 1** | **LED 2** | **LED Behavior** |
|---|---|---|---|---|
| Power on + manual pairing mode | 4 note rising scale | blue | | Continuous flash for 90 seconds (timeout or until connected) 250ms ON, 250ms OFF |
| Power on +automatic paring mode | 4 note rising scale | blue | | Continuous flash for 30 seconds (timeout or until connected) 250ms ON, 250ms OFF. First flash to be displayed at power on |
| Manual pairing mode | 4 note rising scale | blue | | Continuous flash for 90 seconds (timeout or until connected) 250ms ON, 250ms OFF |
| Pairing success | low low high | blue | | Static ON for 5 seconds and flashes once every 30 seconds to show connected |
| Pairing timeout or fail | long low | red | | Static ON for 5 seconds and flashes once every 30 seconds to show not connected |
| Power on, battery 50-100% | 4 note rising scale | | green | Static ON |
| Power on, battery 20-50% | 4 note rising scale + low battery tone | | yellow | Static ON |
| Power on, battery 0-20% | 4 note rising scale + low battery tone | | red | Static ON |
| Power on, battery 0% Earmuff cannot be started | | | red | Three flashes, 100ms ON, 300ms OFF, 100ms ON |
| Phone ringing | ring ... Valkyries | blue | | Rapid (x3 per second) continuous flash until user action 100ms ON - 250ms OFF |
| Clear paired device list and enter manual paring mode | short double low | red + blue | | 23x red and blue alternate flashes 250ms ON/OFF for each color |

Having described various devices and methods herein, exemplary embodiments or aspects can include, but are not limited to:

In a first embodiment, a control interface comprising no more than ten buttons may be configured to receive input from a user via at least one of the no more than ten buttons; control one or more functions of a headset based on the input from the user; and provide feedback to the user based on the received input, wherein the control interface cannot be seen by the user when the user is wearing the headset.

A second embodiment can include the control interface of the first embodiment, wherein the control interface comprises a topography defined by the no more than ten buttons configured to direct a user toward the buttons.

A third embodiment can include the control interface of the first or second embodiments, further comprising an edge defining a perimeter of the control interface and configured to direct a user toward the control interface.

A fourth embodiment can include the control interface of any of the first to third embodiments, further comprising one or more indicators configured to communicate information to the user when the user is not wearing the headset.

A fifth embodiment can include the control interface of any of the first to fourth embodiments, wherein the control interface comprises a power button and one or more system control buttons.

A sixth embodiment can include the control interface of the fifth embodiment, wherein the system control buttons comprise a center button, a left button located to the left of the center button, and a right button located to the right of the center button.

A seventh embodiment can include control interface of the sixth embodiment, wherein the system control buttons further comprises an up button located above the center button, and a down button located below the center button.

An eighth embodiment can include the control interface of the sixth or seventh embodiments, wherein the center button comprises a different topography than the surrounding buttons.

A ninth embodiment can include the control interface of any of the fifth to eighth embodiments, wherein the power button comprises a different topography than one or more system control buttons.

A tenth embodiment can include the control interface of any of the fifth to ninth embodiments, wherein the power button is located separately from the system control buttons.

In an eleventh embodiment, a method for controlling a hearing protection headset (e.g. via a control interface located on the headset - for example using any of the headsets described above) may comprise receiving (e.g. by processor) input (e.g. an input signal) from a user via a control interface comprising no more than ten buttons (or alternatively no more than six buttons or no more than 4 buttons or 4-6 buttons or six buttons or four buttons) (e.g. while the user is wearing the headset (e.g. while the headset is in protective mode) and/or with the user operating the control interface blindly (e.g. by feel and/or without looking at the control interface); controlling (e.g. by processor - sending signal) one or more functions of the headset based on the input from the user (see for example the functions of Table 1, incorporated herein by reference); and providing (e.g. by processor - e.g. sending signal) feedback (see for example Table 2, incorporated herein by reference) to the user based on the received input, wherein the control interface cannot be seen by the user when the user is wearing the headset.

A twelfth embodiment can include the method of the eleventh embodiment, further comprising defining a topography of the control interface by a variation in the elevation of the buttons within the control interface.

A thirteenth embodiment can include the method of the twelfth embodiment, wherein defining the topography of the control interface comprises locating a power button separately from one or more system control buttons.

A fourteenth embodiment can include the method of any of the eleventh to thirteenth embodiments, further comprising defining a perimeter of the control interface by a frame located around the control interface.

A fifteenth embodiment can include the method of any of the eleventh to fourteenth embodiments, wherein providing feedback to the user comprises providing audio feedback via a speaker of the headset.

In a sixteenth embodiment, a headset may comprise one or more earmuffs configured to provide sound blocking; a headband connecting the earmuffs; and a control interface located on an external surface of at least one of the earmuffs, the control interface comprising no more than ten buttons, and configured to receive input from a user via at least one of the no more than ten buttons; control one or more functions of the headset based on the input from the user; and provide feedback to the user based on the received input, wherein the control interface cannot be seen by the user when the user is wearing the headset.

A seventeenth embodiment can include the headset of the sixteenth embodiment, wherein the no more than ten buttons comprise topographical characteristics configured to identify the buttons for the user.

An eighteenth embodiment can include the headset of the sixteenth or seventeenth embodiments, wherein at least one of the buttons is raised with respect to the surface of the control interface.

A nineteenth embodiment can include the headset of any of the sixteenth to eighteenth embodiments, wherein at least one of the buttons is recessed with respect to the surface of the control interface.

A twentieth embodiment can include the headset of any of the sixteenth to eighteenth embodiments, wherein the buttons comprise a power button located separately from one or more system control buttons.

A twenty-first embodiment can include the devices and/or methods of any of the first to twentieth embodiments, wherein the control interface is configured to complete one or more of the following actions: Power on + enter auto pairing, Power off, Quick pairing mode, Mode change, Answer call, Voice Dialing, Transfer call to external device, Clear paired device list & enter manual pairing, End call, Mute microphone toggle, Reject incoming call, Play, Pause, Next track, Scan track forward, Scan track backward, Previous track, Station search forward, Station scan forward, Station search backward, Station scan backward, Recall presets backward, Recall presets forward, Store station, Delete station, and/or Mute radio toggle.

A twenty-second embodiment can include the devices and/or methods of any of the first to twenty-first embodiments, wherein Power on + enter auto pairing is completed by holding the power button for 2 seconds.

A twenty-third embodiment can include the devices and/or methods of any of the first to twenty-second embodiments, wherein Power off is completed by holding the power button for 2 seconds.

A twenty-fourth embodiment can include the devices and/or methods of any of the first to twenty-third embodiments, wherein Quick pairing mode is completed by pressing the power button two times.

A twenty-fifth embodiment can include the devices and/or methods of any of the first to twenty-fourth embodiments, wherein Mode change is completed by pressing the power button.

A twenty-sixth embodiment can include the devices and/or methods of any of the first to twenty-fifth embodiments, wherein Answer call is completed by pressing the center button.

A twenty-seventh embodiment can include the devices and/or methods of any of the first to twenty-sixth embodiments, wherein Voice dialing is completed by holding the center button for 2 seconds.

A twenty-eighth embodiment can include the devices and/or methods of any of the first to twenty-seventh embodiments, wherein Transfer call to external device is completed by pressing the left button two times.

A twenty-ninth embodiment can include the devices and/or methods of any of the first to twenty-eighth embodiments, wherein Clear paired device list and enter manual pairing is completed by holding the power button for 5 seconds.

A thirtieth embodiment can include the devices and/or methods of any of the first to twenty-ninth embodiments, wherein End call is completed by pressing the center button.

A thirty-first embodiment can include the devices and/or methods of any of the first to thirtieth embodiments, wherein Mute microphone toggle is completed by pressing the right button.

A thirty-second embodiment can include the devices and/or methods of any of the first to thirty-first embodiments, wherein Reject incoming call is completed by pressing the left button.

A thirty-third embodiment can include the devices and/or methods of any of the first to thirty-second embodiments, wherein Play is completed by pressing the center button.

A thirty-fourth embodiment can include the devices and/or methods of any of the first to thirty-third embodiments, wherein Pause is completed by pressing the center button.

A thirty-fifth embodiment can include the devices and/or methods of any of the first to thirty-fourth embodiments, wherein Next track is completed by pressing the right button.

A thirty-sixth embodiment can include the devices and/or methods of any of the first to thirty-fifth embodiments, wherein Scan track forward is completed by holding the right button for 2 seconds.

A thirty-seventh embodiment can include the devices and/or methods of any of the first to thirty-sixth embodiments, wherein Scan track backward is completed by holding the left button for 2 seconds.

A thirty-eighth embodiment can include the devices and/or methods of any of the first to thirty-seventh embodiments, wherein Previous track is completed by pressing the left button.

A thirty-ninth embodiment can include the devices and/or methods of any of the first to thirty-eighth embodiments, wherein Station search forward is completed by pressing the right button.

A fortieth embodiment can include the devices and/or methods of any of the first to thirty-ninth embodiments, wherein Station scan forward is completed by holding the right button for 2 seconds.

A forty-first embodiment can include the devices and/or methods of any of the first to fortieth embodiments, wherein Station search backward is completed by pressing the left button.

A forty-second embodiment can include the devices and/or methods of any of the first to forty-first embodiments, wherein Station scan backward is completed by holding the left button for 2 seconds.

A forty-third embodiment can include the devices and/or methods of any of the first to forty-second embodiments, wherein Recall presets backward is completed by pressing the down button.

A forty-fourth embodiment can include the devices and/or methods of any of the first to forty-third embodiments, wherein Recall presets forward is completed by pressing the up button.

A forty-fifth embodiment can include the devices and/or methods of any of the first to forty-fourth embodiments, wherein Store station is completed by holding the up button for 2 seconds.

A forty-sixth embodiment can include the devices and/or methods of any of the first to forty-fifth embodiments, wherein Delete station is completed by holding the down button for 2 seconds.

A forty-seventh embodiment can include the devices and/or methods of any of the first to forty-sixth embodiments, wherein Mute radio toggle is completed by pressing the center button.

While various embodiments in accordance with the principles disclosed herein have been shown and described above, modifications thereof may be made by one skilled in the art without departing from the spirit and the teachings of the disclosure. The embodiments described herein are representative only and are not intended to be limiting. Many variations, combinations, and modifications are possible and are within the scope of the disclosure. Alternative embodiments that result from combining, integrating, and/or omitting features of the embodiment(s) are also within the scope of the disclosure. Accordingly, the scope of protection is not limited by the description set out above, but is defined by the claims which follow that scope including all equivalents of the subject matter of the claims. Each and every claim is incorporated as further disclosure into the specification and the claims are embodiment(s) of the present invention(s). Furthermore, any advantages and features described above may relate to specific embodiments, but shall not limit the application of such issued claims to processes and structures accomplishing any or all of the above advantages or having any or all of the above features.

Additionally, the section headings used herein are provided for consistency with the suggestions under 37 C.F.R. 1.77 or to otherwise provide organizational cues. These headings shall not limit or characterize the invention(s) set out in any claims that may issue from this disclosure. Specifically and by way of example, although the headings might refer to a "Field," the claims should not be limited by the language chosen under this heading to describe the so-called field. Further, a description of a technology in the "Background" is not to be construed as an admission that certain technology is prior art to any invention(s) in this disclosure. Neither is the "Summary" to be considered as a limiting characterization of the invention(s) set forth in issued claims. Furthermore, any reference in this disclosure to "invention" in the singular should not be used to argue that there is only a single point of novelty in this disclosure. Multiple inventions may be set forth according to the limitations of the multiple claims issuing from this disclosure, and such claims accordingly define the invention(s), and their equivalents, that are protected thereby. In all instances, the scope of the claims shall be considered on their own merits in light of this disclosure, but should not be constrained by the headings set forth herein.

Use of broader terms such as "comprises," "includes," and "having" should be understood to provide support for narrower terms such as "consisting of," "consisting essentially of," and "comprised substantially of." Use of the terms "optionally," "may," "might," "possibly," and the like with respect to any element of an embodiment means that the element is not required, or alternatively, the element is required, both alternatives being within the scope of the embodiment(s). Also, references to examples are merely provided for illustrative purposes, and are not intended to be exclusive.

While several embodiments have been provided in the present disclosure, it should be understood that the disclosed systems and methods may be embodied in many other specific forms without departing from the spirit or scope of the present disclosure. The present examples are to be considered as illustrative and not restrictive, and the intention is not to be limited to the details given herein. For example, the various elements or components may be combined or integrated in another system or certain features may be omitted or not implemented.

Also, techniques, systems, subsystems, and methods described and illustrated in the various embodiments as discrete or separate may be combined or integrated with other systems, modules, techniques, or methods without departing from the scope of the present disclosure. Other items shown or discussed as directly coupled or communicating with each other may be indirectly coupled or communicating through some interface, device, or intermediate component, whether electrically, mechanically, or otherwise. Other examples of changes, substitutions, and alterations are ascertainable by one skilled in the art and could be made without departing from the spirit and scope disclosed herein.

## Claims

1. A control interface (110) comprising no more than ten buttons configured to:
receive input from a user via at least one of the no more than ten buttons;
control one or more functions of a headset (100) based on the input from the user; and
provide feedback to the user based on the received input, wherein the control interface (110) cannot be seen by the user when the user is wearing the headset (100).

2. The control interface of claim 1, wherein the control interface (110) comprises a topography defined by the no more than ten buttons configured to direct a user toward the buttons.

3. The control interface of claim 1, further comprising a frame (108) defining a perimeter of the control interface (110) and configured to direct a user toward the control interface (110).

4. The control interface of claim 1, further comprising one or more indicators configured to communicate information to the user when the user is not wearing the headset.

5. The control interface of claim 1, wherein the control interface comprises a power button (111) and one or more system control buttons (112, 113, and 114).

6. The control interface of claim 5, wherein the system control buttons comprise a center button (113), a left button (112) located to the left of the center button (113), and a right button (114) located to the right of the center button (113).

7. The control interface of claim 6, wherein the system control buttons further comprise an up button (115) located above the center button (113), and a down button (116) located below the center button (113).

8. The control interface of claim 6, wherein the center button (113) comprises a different topography than the surrounding buttons (112, 114).

9. The control interface of claim 5, wherein the power button (111) comprises a different topography than one or more system control buttons (112, 113, and 114).

10. The control interface of claim 5, wherein the power button (111) is located separately from the system control buttons (112, 113, and 114).

11. A method for controlling a hearing protection headset, the method comprising:
receiving input from a user to a control interface comprising no more than ten buttons;
controlling one or more functions of the headset based on the input from the user; and
providing feedback to the user based on the received input, wherein the control interface cannot be seen by the user when the user is wearing the headset.

12. The method of claim 11, further comprising defining a topography of the control interface by a variation in the elevation of the buttons within the control interface.

13. The method of claim 12, wherein defining the topography of the control interface comprises locating a power button separately from one or more system control buttons.

14. The method of claim 11, further comprising defining a perimeter of the control interface by a frame located around the control interface.

15. The method of claim 11, wherein providing feedback to the user comprises providing audio feedback via a speaker of the headset.

## Amended claims

### Amended claims in accordance with Rule 137(2) EPC.

1. A control interface (110) incorporated into a hearing protection headset (100) and located on an external face of the hearing protection headset (100), the control interface (110) comprising:
no more than ten buttons each comprising a raised surface protruding from a surface of the control interface (110) and including:
a power button (111);
a center button (113);
a left button (112) located to the left of the center button (113); and
a right button (114) located to the right of the center button (113), the control interface configured to:
receive input from a user via at least one of the no more than ten buttons;
control one or more functions of the hearing protection headset (100) based on the input from the user; and
provide audio feedback to the user based on the received input.

2. The control interface of claim 1, wherein the control interface (110) comprises a topography defined by the no more than ten buttons configured to direct a user toward the buttons.

3. The control interface of claim 1, further comprising a frame (108) defining a perimeter of the control interface (110) and configured to direct a user toward the control interface (110).

4. The control interface of claim 1, further comprising one or more indicators configured to communicate information to the user when the user is not wearing the headset.

5. The control interface of claim 1, wherein the no more than ten buttons further comprise an up button (115) located above the center button (113), and a down button (116) located below the center button (113).

6. The control interface of claim 1, wherein the center button (113) comprises a different topography than the left button (112) and the right button (114).

7. The control interface of claim 1, wherein the power button (111) comprises a different topography than the center button (113), the left button (112), and the right button (114).

8. The control interface of claim 1, wherein the power button (111) is located separately from the center button (113), the left button (112), and the right button (114).

9. A method for controlling a hearing protection headset, the method comprising:
receiving input from a user to a control interface (110) into a hearing protection headset (100) and located on an external face of the hearing protection headset (100), the control interface (110) comprising no more than ten buttons each comprising a raised surface protruding from a surface of the control interface (110) and including:
a power button (111);
a center button (113);
a left button (112) located to the left of the center button (113); and
a right button (114) located to the right of the center button (113), the control interface;
controlling one or more functions of the hearing protection headset based on the input from the user; and
providing audio feedback to the user based on the received input.

10. The method of claim 9, further comprising defining a topography of the control interface by a variation in the elevation of the buttons within the control interface.

11. The method of claim 9, further comprising defining a perimeter of the control interface by a frame located around the control interface.

12. The method of claim 9, wherein providing the audio feedback to the user comprises providing audio feedback via a speaker of the headset.
